# EUROPÄISCHE PATENTSCHRIFT

(11) **EP 0 993 430 B1**
(45) Veröffentlichungstag und Bekanntmachung des Hinweises auf die Patenterteilung: **11.09.2002**
(21) Anmeldenummer: 98938622.2
(22) Anmeldetag: 22.06.1998
(51) Int. Cl.: C07C 37/74, C07C 37/14, C07C 37/52, C07C 39/17

(54) **HERSTELLUNG UND REINIGUNG VON 3-(4-HYDROXYPHENYL)-1,1,3-TRIMETHYLINDAN-5-OL**
METHOD FOR PREPARING AND PURIFYING 3-(4-HYDROXYPHENYL)-1,1,3-TRIMETHYLINDAN-5-OL
PROCEDE DE PREPARATION ET D'EPURATION DE 3-(4-HYDROXYPHENYL)-1,1,3-TRIMETHYLINDAN-5-OL

(30) Priorität: 03.07.1997 DE 19728379
(43) Veröffentlichungstag der Anmeldung: 19.04.2000
(73) Patentinhaber: BAYER AG, 51368 Leverkusen (DE)
(72) Erfinder: WESTERNACHER, Stefan, Seabrook, TX 77586 (US)
(86) Internationale Anmeldenummer: EP9803803
(87) Internationale Veröffentlichungsnummer: WO99001414

(56) Entgegenhaltungen:
- US-A- 3 288 864
- US-A- 4 334 106
- US-A- 4 366 328
- DATABASE WPI Section Ch, Week 7532 Derwent Publications Ltd., London, GB; Class E14, AN 75-53040W XP002081238 & JP 50 035150 A (MITSUI TOATSU CHEM INC) , 3. April 1975 in der Anmeldung erwähnt
- DATABASE WPI Section Ch, Week 7930 Derwent Publications Ltd., London, GB; Class A60, AN 79-55597B XP002081239 & JP 54 076564 A (MITSUI TOATSU CHEM INC) , 19. Juni 1979
- DATABASE WPI Section Ch, Week 7830 Derwent Publications Ltd., London, GB; Class A41, AN 78-54215A XP002081240 & JP 53 068762 A (AGENCY OF IND SCI & TECHNOLOGY), 19. Juni 1978

## Beschreibung

Die Erfindung betrifft ein Verfahren zur Herstellung und Reinigung von 3-(4-Hydroxyphenyl)-1,1,3-trimethylindan-5-ol durch Isomerisierung von Isopropenylphenol, dessen Dimeren oder Oligomeren und anschließende Destillation des Reaktionsgemischs.

Für die Herstellung von 3-(4-Hydroxyphenyl)-1,1,3-trimethylindan-5-ol, im folgenden Bisphenol-Indan genannt, sind bereits verschiedene Verfahren bekannt geworden.

So offenbaren US-A 2 754 285 und US-A 2 819 249 einen Herstellungsweg über die säurekatalysierte Dimerisierung von α-Methylstyrol zu Indanen, welche anschliessend sulfoniert und dann mit Kaliumhydroxid verseift werden.

US-A 2 979 534 lehrt, daß die durch Spaltung von Bisphenolen erhaltenen monomeren Isopropenylphenole in Gegenwart aromatischer Sulfonsäuren oder Mineralsäuren bei Temperaturen von 110 bis 160°C zu Bisphenol-Indan dimerisiert werden können. Bisphenolspaltung und Indanbildung können auch in einem Schritt durchgeführt werden. Nach diesem Verfahren wurde ein Produkt geringer Reinheit erhalten, das auch nach Rekristallisation aus Benzol/Cyclohexan nur einen Schmelzpunkt von 165-166°C aufwies.

US-A 3 264 357 offenbart die Herstellung von Bisphenolen durch Reaktion einer Mischung der beiden isomeren Formen des dimeren Isopropenylphenols mit Phenolen in Gegenwart starker Säuren. Es wird berichtet, daß in Abwesenheit reaktiver Phenole bei einer Reaktionstemperatur von 90°C Bisphenol-Indan gebildet wird. Nach USA 3 264 358 kann Bisphenol-Indan durch Umsetzung einer Mischung der beiden isomeren Formen des dimeren Isopropenylphenols mit stark sauren Katalysatoren erhalten werden, beispielsweise durch zweistündiges Tempern in konzentrierter Salzsäure bei Siedehitze.

US-A 3 288 864 offenbart die Herstellung von Bisphenol-Indan durch Selbstkondensation von monomerem Isopropenylphenol in Gegenwart von Friedel-Crafts-Katalysatoren bei Temperaturen von 50 bis 150°C, JP-A 60/35150 lehrt die Isomerisierung von Isopropenylphenol oder dessen Oligomeren in Gegenwart fester Katalysatoren wie Aluminiumoxid oder Terra alba.

Nach US-A 4 334 106 kann Bisphenol-Indan durch Umsetzung von Isopropenylphenol oder dessen Oligomeren in Halogencarbonsäuren oder Ameisensäure bei Temperaturen von 0 bis 90°C hergestellt werden.

Gemäß JP-A 5/294879 kann Bisphenol-Indan durch thermische Zersetzung von Bisphenol-A in Gegewart von aktiviertem Ton erhalten werden, US-A 3 271 463 offenbart die Bildung von Bisphenol-Indan als Nebenprodukt bei der Behandlung von Bisphenol A mit wäßriger Schwefelsäure bei 90-150°C. Bei beiden Verfahren werden größere Mengen an Spirobisindan-Bisphenol gebildet, welches durch Umkristallisation aus aromatischen Kohlenwasserstoffen abgetrennt werden muß.

Die beschriebenen Verfahren sind für eine industrielle Produktion von Bisphenol-Indan zur Verwendung als Ausgangsstoff für die Kunststoffherstellung vielfach noch unbefriedigend. Um Bisphenol-Indan in der hierfür erforderlichen Reinheit zu erhalten, ist es notwendig, das Reaktionsprodukt in aufwendiger Weise aus dem Reaktionsgemisch abzutrennen und durch Umkristallisation zu reinigen. Es wurde nun ein Verfahren gefunden, das diese Schritte verzichtbar macht.

Gegenstand der Erfindung ist ein Verfahren zur Herstellung und Reinigung von Bisphenol-Indan, bei dem man Isopropenylphenol, dessen Dimere oder Oligomere oder deren Mischungen in Gegenwart eines sauren Katalysators isomerisiert, die Reaktionsmischung durch Zugabe einer Base neutralisiert und anschließend aus dem Reaktionsgemisch durch Destillation (z.B. fraktionierende Destillation) Bisphenol-Indan isoliert.

Als Ausgangsstoffe für das erfindungsgemäße Verfahren werden Isopropenylphenol, dessen Dimere oder Oligomere oder deren Mischungen eingesetzt. Diese sind leicht zugänglich und können beispielsweise nach den in US-A 3 288 864 oder USA 4 201 877 beschriebenen Methoden hergestellt werden.

Die Isomerisierung wird bevorzugt in Gegenwart eines Lösungsmittels durchgeführt. Als Lösungsmittel ist eine Vielzahl unterschiedlicher Lösungsmittel geeignet. Beispiele sind Kohlenwasserstoffe wie Petroläther, Cyclohexan, Benzol, Toluol oder Xylol, Alkohole wie Methanol, n-Propanol oder n-Butanol, Carbonsäuren wie Ameisensäure oder Essigsäure, Propionsäure, Halogencarbonsäuren wie Trichloressigsäure oder Trifluoressigsäure, chlorierte Kohlenwasserstoffe wie Methylenchlorid, Chloroform, Trichlorethylen oder Tetrachlorkohlenstoff, substituierte Aromaten wie Chlorbenzol oder Nitrobenzol; ebenfalls geeignet sind Dimethylsulfoxid, Dimethylformamid, Dimethylacetamid oder N-Methylpyrrolidon. Bevorzugt wird Chlorbenzol verwendet. Die Menge an Lösungsmittel beträgt bevorzugt das zwei- bis dreifache der eingesetzten Menge an Isopropenylphenol.

Die Isomerisierungsreaktion wird in Gegenwart eines sauren Katalysators durchgeführt. Als Katalysatoren für das erfindungsgemäße Verfahren können Brönstedt- oder Lewis-Säuren verwendet werden. Beispiele sind Mineralsäuren wie Salzsäure oder Schwefelsäure, organische Säuren wie Sulfonsäuren oder Halogencarbonsäuren, Bortrifluorid und Metallhalogenide wie AlCl₃, FeCl₃ oder ZnCl₂. Daneben können auch heterogene Katalysatorsysteme eingesetzt werden, z.B. in Form eines Festbetts. Beispiele sind saure Ionentauscherharze, Zeolithe, Oxide oder Hydroxide oder Mischoxide von Übergangsmetallen oder seltenen Erden, Heteropolysäuren, Al₂O₃, SiO₂ und deren Mischungen. Bevorzugt werden als Katalysatoren Lewis-Säuren eingesetzt, besonders bevorzugt Bortrifluorid. In einer bevorzugten Ausrührungsform des Verfahrens wird der Katalysator der Reaktionsmischung nach Erwärmen auf eine Temperatur im Bereich von 60 bis 110°C, bevorzugt 70 bis 90°C, in einer Menge von 0,002 bis 5 Gew.-%, bevorzugt 0,3 bis 0,5 Gew.-%, bezogen auf die eingesetzte Menge an Isopropenylphenol, gegebenenfalls portionsweise zugesetzt.

Die Isomerisierung wird bevorzugt bei einer Temperatur im Bereich von 0 bis 160°C durchgeführt, besonders bevorzugt bei einer Temperatur im Bereich von 110 bis 150°C, ganz besonders bevorzugt bei 130 bis 140°C. Man läßt bevorzugt für 1 bis 600 Minuten, besonders bevorzugt für 2 bis 60 Minuten reagieren.

Im Anschluß an die Isomerisierungsreaktion wird die Reaktionsmischung gegebenenfalls durch Zusatz einer Base neutralisiert. Die Neutralisation erfolgt bevorzugt bei einer Temperatur im Bereich von 60 bis 100°C, besonders bevorzugt 70 bis 90°C. Zur Neutralisation ist eine Vielzahl verschiedener Basen oder deren Mischungen geeignet. Beispiele sind Metallhydroxide wie NaOH, KOH, Mg(OH)₂, Ca(OH)₂, Alkoholate wie Natriummethanolat, Natriumethanolat, Natriumphenolat, Kaliummethanolat, Kaliumethanolat, Kaliumphenolat, Magnesiummethanolat, Magnesiumethanolat, Magnesiumphenolat, Calciummethanolat, Calciumethanolat, Calciumphenolat, Aluminiumisopropylat, Carboxylate wie Natriumformiat, Natriumacetat, Natriumbenzoat, Calciumformiat, Calciumacetat, Carbonate wie Natriumcarbonat, Kaliumcarbonat, Calciumcarbonat, Magnesiumcarbonat, (NH₄)₂CO₃, Hydrogencarbonate wie NaHCO₃, KHCO₃ oder NH₄HCO₃, Mischungen aus NH₄HCO₃ und Ammoniumcarbonat, Ammoniak, Amine wie Triethylamin, Diethylamin, Ethylamin, Trimethylamin, Dimethylamin, Methylamin und deren Lösungen in Wasser oder organischen Lösungsmitteln, die nicht mit dem Reaktionsmedium mischbar sind. Bevorzugt wird als Base wäßrige Natronlauge verwendet. Wird eine Base verwendet, die mit dem Reaktionsgemisch ein Zweiphasensystem bildet, so wird nach der Neutralisation die das Bisphenol-Indan enthaltende organische Phase abgetrennt.

Aus der neutralisierten Reaktionsmischung bzw. der das Bisphenol-Indan enthaltenden organischen Phase wird anschließend durch fraktionierende Destillation Bisphenol-Indan isoliert. Obwohl US-A 4 366 328 lehrt, daß sich Bisphenol-Indane schon bei Temperaturen ab 150°C unter Bildung der korrespondierenden Indene zersetzen, wobei die Zersetzungsreaktion unter vermindertem Druck beschleunigt abläuft, gelingt es, aus dem Reaktionsgemisch durch Destillation Bisphenol-Indan hoher Reinheit in guter Ausbeute zu erhalten. Hierbei werden zunächst leichtsiedende Anteile (z.B. Lösungsmittel und nicht umgesetztes Edukt) entfernt, anschließend wird Bisphenol-Indan abdestilliert. Dies erfolgt bevorzugt bei Drücken im Bereich von 10⁻³ bis 10¹ mbar und Temperaturen von 160 bis 230°C, besonders bevorzugt bei 10⁻² bis 10⁰ mbar und 175 bis 205°C. Die Abtrennung der Leichtsieder und die Destillation des Bisphenols können in einem einzigen Schritt oder auch nacheinander durchgeführt werden. Alternativ kann die Trennung beispielsweise in zwei hintereinandergeschalteten Destillationskolonnen erfolgen, wobei die Hochsieder am Fuß der ersten Kolonne und die Leichtsieder als Kopfprodukt der zweiten Kolonne erhalten werden, während Bisphenol-Indan am Fuß der zweiten Kolonne entnommen wird. Bevorzugt wird die Destillation unter Inertgasatmosphäre, z.B. Stickstoff oder Argon, durchgeführt. Die Reaktionsmischung wird zweckmäßigerweise vor der Destillation durch Einleiten von Inertgas von Luftsauerstoff befreit.

Aufgrund seiner hohen Reinheit ist das nach dem erfindungsgemäßen Verfahren hergestellte Bisphenol-Indan in hervorragender Weise geeignet als Ausgangsstoff für die Herstellung von hochwertigen Kunststoffen, z.B. Polycarbonaten.

### Beispiel

800 g dimeres Isopropenylphenol wurden in 2000 ml Chlorbenzol gelöst. Nach Erwärmen der Reaktionsmischung auf 80°C wurden 2,8 ml BF₃ als Etherat zugegeben. Die Reaktionslösung wurde zum Sieden erhitzt und dann für 40 Minuten bei 132°C unter Rückfluß gehalten. Anschließend wurde der Katalysator in der Reaktionslösung durch Zugabe von Natronlauge (1.164 g NaOH auf 300 ml Wasser) bei 80°C neutralisiert. Die wäßrige Phase wurde abgetrennt, die organische Phase der Destillation zugeführt. Bei einem Druck von 1 mbar wurden zunächst Lösungsmittel und nicht umgesetztes Edukt abdestilliert. Schließlich wurden bei 198°C Kopftemperatur 440 g (entsprechend 55 % der Einwaage an Isopropenylphenol) einer Fraktion mit einem Bisphenol-Indan-Gehalt von 92 % isoliert.

## Patentansprüche

1. Verfahren zur Herstellung und Reinigung von 3-(4-Hydroxyphenyl)-1,1,3-trimethylindan-5-ol, bei dem man
a) Isopropenylphenol, dessen Dimere oder Oligomere oder deren Mischungen in Gegenwart eines sauren Katalysators isomerisiert,
b) die Reaktionsmischung durch Zugabe einer Base neutralisiert,
c) aus dem Reaktionsgemisch durch Destillation 3-(4-Hydroxyphenyl)-1,1,3-trimethylindan-5-ol isoliert.

## Claims

1. Process for the preparation and purification of 3-(4-hydroxyphenyl)-1,1,3-trimethylindan-5-ol, wherein
a) isopropenylphenol, its dimers or oligomers or mixtures thereof are isomerised in the presence of an acid catalyst,
b) the reaction mixture is neutralised by the addition of a base,
c) 3-(4-hydroxyphenyl)-1,1,3-trimethylindan-5-ol is subsequently isolated from the reaction mixture by fractional distillation.

## Revendications

1. Procédé de préparation et de purification de 3-(4-hydroxyphényl)-1,1,3-triméthylindan-5-ol, où l'on
a) isomérise de l'isopropénylphénol, ses dimères ou oligomères ou leurs mélanges en présence d'un catalyseur acide,
b) neutralise le mélange réactionnel par addition d'une base,
c) isole le 3-(4-hydroxyphényl)-1,1,3-triméthylindan-5-ol du mélange réactionnel par distillation.
